# EUROPEAN PATENT SPECIFICATION

(11) **EP 2 906 203 B1**
(45) Date of publication and mention of the grant of the patent: **03.01.2018**
(21) Application number: 13782998.2
(22) Date of filing: 10.10.2013
(51) Int. Cl.: A61K 9/46, A61K 9/00

(54) **EFFERVESCENT CEFDINIR FORMULATION**
BRAUSEFORMULIERUNG VON CEFDINIR
FORMULATION EFFERVESCENTE DE CEFDINIR

(30) Priority: 11.10.2012 TR 201211647
(43) Date of publication of application: 19.08.2015
(73) Proprietor: Sanovel Ilac Sanayi Ve Ticaret Anonim Sirketi, 34460 Istanbul (TR)
(72) Inventor: TÜRKYILMAZ, Ali, 34460 Istanbul (TR); FILIZ, Cigdem, 34460 Istanbul (TR)
(74) Representative: Sevinç, Erkan
(86) International application number: PCT/EP2013/071207
(87) International publication number: WO 2014/057059

(56) References cited:
- WO-A1-2011/078830
- WO-A1-2011/152806
- CN-A- 1 706 389

## Description

### Field of Invention

The present invention relates to formulations of cefdinir or a pharmaceutically acceptable salt of cefdinir. The present invention more particularly relates to effervescent formulations enabling to increase the dissolution rate and solubility of cefdinir.

### Background of Invention

Cefdinir with the chemical name (6R,7R)-7-[(Z)-2-(2-aminothiazol-4-yl)-2-hydroxy-iminoglioxylamido]-8-oxo-3-vinyl-5-thia-1-azabicyclo[4.2.0]oct-2-ene-2-carbonic acid is a broad-spectrum semi-synthetic cephalosporin. The chemical structure of cefdinir is illustrated in Formula I given below.

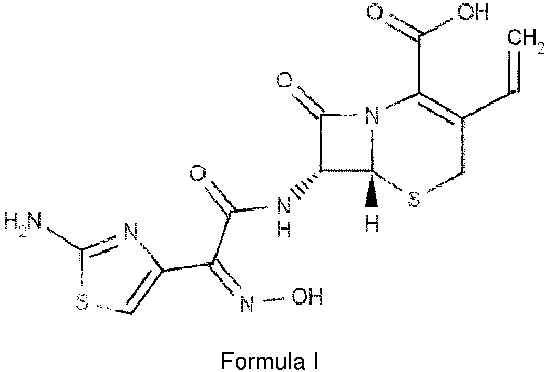

Cefdinir was originally disclosed in the application US 4,559,334.

The patent application EP 1 905 432 discloses a stable nanoparticulate composition, comprising cephalosporin with a diameter size less than 2000 nm and at least one surface stabilizer adsorbed on the surface of particles, said surface stabilizer being free of intermolecular cross-linkages.

The patent application WO2005060936 claims an oral suspension formulation comprising cefdinir.

The patent EP0890359B1 claims a tablet formulation comprising beta-lactam antibiotics including cefdinir among the others. According to that formulation, a tablet comprises 60 to 85% by weight of a beta-lactam antibiotic and 1 to 10% by weight of hydroxypropyl cellulose and/or cross-linked polyvinylpyrrolidone, hydroxypropyl cellulose, or hydroxypropyl methylcellulose.

The patent US2006233878 discloses a controlled-release formulation comprising a beta-lactam antibiotic and one or more carbomer(s).

WO 2011/152806 A1 discloses effervescent compositions comprising a cephalosporin antibiotic, such as cefdinir and clavulanic acid. If the cephalosporin antibiotic and clavulanic acid are mixed with the granules comprising effervescent couple and at least one excipient in the presence of high-molecular weight PEG, gelling and agglomeration problem are eliminated. Excipients can be e.g. PVP as binder; glidants, such as sodium benzoate, L-leucine, PEG or a combination thereof.

Cefdinir is a solid having with a white or whitish yellow color. Practically, it is water-insoluble. Its lower solubility and dissolution rate reduce the bioavailability of cefdinir.

### Content Uniformity of the Formulation:

A cefdinir formulation comprising any dosage amount for use, for instance, should comprise the same amount of cefdinir in all identical cefdinir formulations. The selection and the amount of excipients in each dosage form directly affect some parameters such as the disintegration, the stability, the solubility, and the dissolution rate of the formulation. The content uniformity of cefdinir formulations is important in providing an efficient treatment. Content uniformity may be achieved using suitable excipients and suitable processes. The lack of providing content uniformity causes different units of an administered cefdinir composition to provide different treatment levels. This, in turn, is not desired in terms of users.

### Flowability:

A cefdinir composition not adhering to machinery and equipment, as well as a flowable powder form prior to tablet compression facilitate the production process.

### Disintegration/Solubility/Dissolution Rate

Many cefdinir formulations should disintegrate and dissolve well enough and quickly in drink solutions in which they are used and should have an excellent dissolution rate. Thus, high levels of bioavailability can be reached. Solving all such problems depends on the suitability of excipients to be selected. For instance, the selection of a lubricant is important beside the selected disintegrants. Whilst it is a good lubricant, magnesium stearate has some known drawbacks and therefore it is used in drug production in small amounts. Magnesium stearate is almost insoluble in water and due to this hydrophobic character, the dissolution of a drug from a solid dosage form such as a tablet or capsule may be retarded. The dissolution of a tablet and particularly of a capsule depends both on the amount of magnesium stearate present in the formulation and to the blending time thereof. The blending time should be limited. Long blending times may result in the formation of hydrophobic powder beds in the formulation which do not dissolve easily, whereas overblending can cause compaction problems. Tablet dissolution rate and crushing strength decrease as the time of blending increases, and magnesium stearate may also increase the tablet friability. For this reason, blending times with magnesium stearate should be controlled carefully. (Handbook of Pharmaceutical Excipients, 5th Edition, Rowe, Raymond C., Sheskey, Paul J., Owen, Sian C., pages 430-432)*.*

### Stability

Various stability problems are encountered in cefdinir formulations due to ambient and physical conditions. Cefdinir formulations are considerably affected from temperature, air, and humidity conditions. Exposure to air and humidity causes structural degradation and to develop behavioral changes. Therefore, the stability of such formulations turns out to be poor and the shelf life thereof becomes shortened. In addition, such active agents are reactive against the excipients employed in developing the formulations containing the same. This, in turn, causes impurities to occur in the formulations and leads to the inclusion of undesired components into the formulations. It is of critical importance in terms of the formulation to use those excipients and methods which do not lead to said problems.

In result, a novelty is needed in the art of cefdinir formulations to provide the advantages of content uniformity, production convenience, as well as satisfactory solubility, dissolution rate, and stability.

Currently available formulations require a certain period of time to achieve efficiency. Considering this problem, it is obvious that a novelty is needed in the relevant art of formulations comprising cefdinir.

### Object and Brief Description of Invention

The present invention provides an effervescent cefdinir formulation eliminating all aforesaid problems and bringing additional advantages to the relevant prior art.

Accordingly, the main object of the present invention is to obtain a stable effervescent cefdinir formulation with improved bioavailability.

Another object of the present invention is to obtain a formulation by which the solubility and the dissolution rate of cefdinir are enhanced.

By virtue of the formulation as claimed in the paragraph above, a further object of the present invention is to eliminate the problems related to decreasing flowability during production and to adhesion to equipment used in the production.

A pharmaceutical effervescent formulation of cefdinir or a pharmaceutically acceptable salt or polymorph of cefdinir has been developed to carry out all objects, referred to above and to emerge from the following detailed description.

According to a preferred embodiment of the present invention, said novelty is characterized by using at least two lubricants, wherein one of said lubricants is polyethylene glycol and wherein the other lubricant is selected from sodium stearyl fumarate, leucine, poloxamer, sodium benzoate, sodium lauryl sulfate or a proper mixture thereof and said formulation does not comprise magnesium stearate. The ratio by weight of cefdinir to the lubricants is between 1:10 and 10:1.

In a preferred embodiment according to the present invention, the ratio by weight of cefdinir to the lubricants is between 1:10 and 8:1.

In a preferred embodiment according to the present invention, the ratio by weight of cefdinir to the lubricants is between 1:10 and 6:1.

In another preferred embodiment according to the present invention, said formulation is obtained by means of a slug compression method not involving any liquid solvent during the granulation step.

In a further preferred embodiment according to the present invention, the average molecular weight of polyethylene glycol is in the range of 7000 to 9000.

Another preferred embodiment according to the present invention further comprises a binder.

In another preferred embodiment according to the present invention, said binder is preferably polyvinylpyrrolidone.

In a further preferred embodiment according to the present invention, the polyvinylpyrrolidone is a K-30 type polyvinylpyrrolidone. The K value calculated according to the Fikentscher equation is between 27 and 33.

In another preferred embodiment according to the present invention, said lubricants are preferably sodium stearyl fumarate and polyethylene glycol, respectively.

In another preferred embodiment according to the present invention, said lubricants are poloxamer and polyethylene glycol, respectively.

In another preferred embodiment according to the present invention, said lubricants are sodium lauryl sulfate and polyethylene glycol, respectively.

Still another preferred embodiment according to the present invention further comprises a disintegrant. The disintegrant is selected from sodium starch glycolate, calcium carboxymethyl cellulose, and croscarmellose sodium.

A further preferred embodiment according to the present invention consisting of,
a. 50 to 90% by weight of cefdinir or a pharmaceutically acceptable salt thereof,
b. 5 to 30% by weight of citric acid,
c. 10 to 60% by weight of sodium hydrogen carbonate,
d. 0.1 to 5% by weight of polyvinylpyrrolidone,
e. 0.1 to 5% by weight of polyethylene glycol,
f. 0.1 to 3% by weight of sodium stearyl fumarate,
g. 1 to 5% by weight of orange flavor,
h. 1 to 5% by weight of sucralose.

### Detailed Description of Invention

### Example 1

| **Ingredients** | % |
|---|---|
| Cefdinir | 50- 90 |
| Citric acid | 5 - 30 |
| Sodium hydrogen carbonate | 10 - 60 |
| Polyvinylpyrrolidone | 0,1 - 5 |
| Polyethylene glycol | 0,1 - 5 |
| Sodium stearyl fumarate | 0,1 - 3 |
| Orange flavor | 1 - 5 |
| Sucralose | 1 - 5 |

### Example 2

| **Ingredients** | % |
|---|---|
| Cefdinir | 50 - 90 |
| Citric acid | 5 - 30 |
| Sodium hydrogen carbonate | 10 - 60 |
| Polyvinylpyrrolidone | 0,1 - 5 |
| Polyethylene glycol | 0,1 - 5 |
| Leucine | 0,1 - 3 |
| Orange flavor | 1 - 5 |
| Sucralose | 1 - 5 |

### Example 3

| **Ingredients** | % |
|---|---|
| Cefdinir | 50 - 90 |
| Citric acid | 5 - 30 |
| Sodium hydrogen carbonate | 10 - 60 |
| Polyvinylpyrrolidone | 0,1 - 5 |
| Polyethylene glycol | 0,1 - 5 |
| Poloxamer | 0,1 - 3 |
| Orange flavor | 1 - 5 |
| Sucralose | 1 - 5 |

### Example 4

| **Ingredients** | % |
|---|---|
| Cefdinir | 50 - 90 |
| Citric acid | 5 - 30 |
| Sodium hydrogen carbonate | 10 - 60 |
| Low-substituted hydroxypropyl cellulose | 0,1 - 5 |
| Polyethylene glycol | 0,1 - 5 |
| Sodium lauryl sulfate | 0,5 - 3 |
| Orange flavor | 1 - 5 |
| Sucralose | 1 - 5 |

### Example 5

| **Ingredients** | % |
|---|---|
| Cefdinir | 50 - 90 |
| Citric acid | 5 - 30 |
| Sodium hydrogen carbonate | 10 - 60 |
| Low-substituted hydroxypropyl cellulose | 0,1 - 5 |
| Polyethylene glycol | 0,1 - 5 |
| Sodium benzoate | 0,5 - 3 |
| Orange flavor | 1 - 5 |
| Sucralose | 1 - 5 |

The formulations according to the present invention are prepared as follows. Cefdinir, citric acid, sodium hydrogen phosphate, PVPK 30, 1/3^{rd} of PEG 8000, ½ of any of sodium stearyl fumarate, leucine, poloxamer, sodium benzoate, sodium lauryl sulfate or a proper mixture thereof, orange flavor, and sucralose are weighed as the inner phase and passed through a compactor. The resulting compacted powder is removed from the compactor and an outer phase is added thereto, comprising the remainder 2/3^{rd} of PEG 8000 and the remainder ½ of any of sodium stearyl fumarate, leucine, poloxamer, sodium benzoate, sodium lauryl sulfate, or a proper mixture thereof. The resulting mixture is compressed into tablets.

The formulation developed according to the present invention and that the maximum average particle size of cefdinir in this formulation is 10 µm

With the present invention realized, stable cefdinir formulations can be obtained which have surprisingly good solubility and dissolution rates, and thus have a high bioavailability.

The difficulty of compressing active agents with a small particle size into granules is known. The pharmaceutical composition according to the present invention overcomes the problems encountered in the methods according to the prior art and thus provides a pharmaceutical cefdinir composition free of magnesium stearate; and by using any of sodium stearyl fumarate, leucine, poloxamer, sodium lauryl sulfate or a proper mixture thereof together with polyethylene glycol provides an effervescent cefdinir formulation which has a surprisingly high solubility and dissolution rate, which is considerably clear and does not leave any residues. In addition, the pharmaceutical composition according to the present invention avoids the problem by which powders or granules adhere to tablet compression equipment during production and granule/tablet compression processes. This, in turn, is achieved by keeping the ratio by weight of cefdinir to the lubricants in between 1:10 and 10:1. The polyethylene glycol used according to the present invention is preferably PEG8000. Additionally, the binder used in the slug compaction process according to the present invention is preferably polyvinylpyrrolidone. The most preferable binder in this formulation is polyvinylpyrrolidone. Besides the use as a binder in the slug compaction process, this binder also positively affects the disintegration, solubility, dissolution rate, and the flowability of the product. The polyvinylpyrrolidone is preferably PVP K 30.

The present invention provides a pharmaceutical composition which has high bioavailability, improved solubility and dissolution rate and is stable throughout the shelf life thereof.

The formulation according to the present invention is used in the treatment of bronchitis, pharyngitis, otitis media, pneumonia, sinusitis, tonsillitis, bacterial vaginosis, and infectious tissue diseases.

## Claims

1. An effervescent pharmaceutical formulation of cefdinir or a pharmaceutically acceptable salt or polymorph thereof, comprising at least two lubricants,
- wherein one of said lubricants is polyethylene glycol and the other lubricant is selected from sodium stearyl fumarate, leucine, poloxamer, sodium benzoate, sodium lauryl sulfate or a proper mixture thereof and the ratio by weight of cefdinir to the lubricants is in the range of 1:10 to 10:1, and
- said formulation does not comprise magnesium stearate.

2. The pharmaceutical formulation according to claim 1, wherein the ratio by weight of cefdinir to the lubricants is preferably in the range of 1:10 to 8:1.

3. The pharmaceutical formulation according to any of the preceding claims, wherein the ratio by weight of cefdinir to the lubricants is more preferably in the range of 1:10 to 6:1.

4. The pharmaceutical formulation according to any of the preceding claims, wherein the formulation is obtained by means of a slug compression method not involving any liquid solvent during the granulation step.

5. The pharmaceutical formulation according to any of the preceding claims, wherein the average molecular weight of polyethylene glycol is in the range of 7000 to 9000.

6. The pharmaceutical formulation according to any of the preceding claims, further comprising a binder.

7. The pharmaceutical formulation according to any of the preceding claims, wherein the binder is preferably polyvinylpyrrolidone.

8. The pharmaceutical formulation according to any of the preceding claims, wherein the K value of polyvinylpyrrolidone is 30.

9. The pharmaceutical formulation according to any of the preceding claims, wherein the lubricants are preferably sodium stearyl fumarate and polyethylene glycol, respectively.

10. The pharmaceutical formulation according to any of the preceding claims, wherein the lubricants are poloxamer and polyethylene glycol, respectively.

11. The pharmaceutical formulation according to any of the preceding claims, wherein the lubricants are sodium lauryl sulfate and polyethylene glycol, respectively.

12. The pharmaceutical formulation according to any of the preceding claims, further comprising a disintegrant.

13. The pharmaceutical formulation according to any of the preceding claims, consisting of
a. 50 to 90% by weight of cefdinir or a pharmaceutically acceptable salt thereof,
b. 5 to 30% by weight of citric acid,
c. 10 to 60% by weight of sodium hydrogen carbonate,
d. 0.1 to 5% by weight of polyvinylpyrrolidone,
e. 0.1 to 5% by weight of polyethylene glycol,
f. 0.1 to 3% by weight of sodium stearyl fumarate,
g. 1 to 5% by weight of orange flavor,
h. 1 to 5% by weight of sucralose.

14. The pharmaceutical formulation according to any of the preceding claims for use in preventing or treating bronchitis, pharyngitis, otitis media, pneumonia, sinusitis, tonsillitis, bacterial vaginosis, and infectious tissue diseases in mammalians and particularly in humans.

## Patentansprüche

1. Eine sprudelnde pharmazeutische Rezeptur von Cefdinir oder einem pharmazeutisch akzeptierbaren Salz oder Polymorphen davon, umfassend mindestens zwei Gleitmittel,
- wobei eines der Gleitmittel Polyethylenglycol ist und das andere Gleitmittel ausgewählt ist aus Natriumstearylfumarat, Leucin, Poloxamer, Natriumbenzoat, Natriumlaurylsulfat oder einer entsprechenden Mischung davon und das Gewichtsverhältnis von Cefdinir zu den Gleitmitteln in dem Bereich von 1:10 zu 10:1 ist, und
- die Formulierung nicht Magnesiumstearat umfasst.

2. Pharmazeutische Rezeptur gemäß Anspruch 1, wobei das Gewichtsverhältnis von Cefdinir zu den Gleitmitteln bevorzugt in dem Bereich von 1:10 zu 8:1 ist.

3. Pharmazeutische Rezeptur gemäß einem der vorhergehenden Ansprüche, wobei das Gewichtsverhältnis von Cefdinir zu den Gleitmitteln noch bevorzugter in dem Bereich von 1:10 zu 6:1 ist.

4. Pharmazeutische Rezeptur gemäß einem der vorhergehenden Ansprüche, wobei die Formulierung mittels eines Schneckenkompressionsverfahrens erhalten wird, welches kein flüssiges Lösungsmittel während des Granulierungsschritts involviert.

5. Pharmazeutische Rezeptur gemäß einem der vorhergehenden Ansprüche, wobei das mittlere Molekulargewicht des Polyethylenglycols in den Bereich von 7000 to 9000 ist.

6. Pharmazeutische Rezeptur gemäß einem der vorhergehenden Ansprüche, des Weiteren ein Bindemittel umfassend.

7. Pharmazeutische Rezeptur gemäß einem der vorhergehenden Ansprüche, wobei das Bindemittel bevorzugt Polyvinylpyrrolidon ist.

8. Pharmazeutische Rezeptur gemäß einem der vorhergehenden Ansprüche, wobei der K-Wert von Polyvinylpyrrolidon 30 ist.

9. Pharmazeutische Rezeptur gemäß einem der vorhergehenden Ansprüche, wobei die Gleitmittel jeweils bevorzugt Natriumstearylfumarat und Polyethylenglycol sind.

10. Pharmazeutische Rezeptur gemäß einem der vorhergehenden Ansprüche, wobei die Gleitmittel jeweils Poloxamer und Polyethylenglycol sind.

11. Pharmazeutische Rezeptur gemäß einem der vorhergehenden Ansprüche, wobei die Gleitmittel jeweils Natriumlaurylsulphat und Polyethylenglycol sind.

12. Pharmazeutische Rezeptur gemäß einem der vorhergehenden Ansprüche, des Weiteren ein Sprengmittel umfassend.

13. Pharmazeutische Rezeptur gemäß einem der vorhergehenden Ansprüche, bestehend aus
a. 50 bis 90 Gew% von Cefdinir oder eines pharmazeutisch akzeptierbaren Salzes davon,
b. 5 bis 30 Gew% Zitronensäure,
c. 10 bis 60 Gew% Natriumhydrogencarbonat,
d. 0,1 bis 5 Gew% Polyvinylpyrrolidon,
e. 0,1 bis 5 Gew% Polyethylenglycol,
f. 0,1 bis 3 Gew% Natriumstearylfumarat,
g. 1 bis 5 Gew% Orangengeschmack,
h. 1 bis 5 Gew% Sucralose.

14. Pharmazeutische Rezeptur gemäß einem der vorhergehenden Ansprüche zur Verwendung in der Vorbeugung oder Behandlung von Bronchitis, Pharyngitis, Mittelohrentzündung, Pneumonia, Sinusitis, Tonsilitis, bakterieller Vaginose und infektiösen Gewebeerkrankungen bei Säugetieren, insbesondere beim Menschen.

## Revendications

1. - Formulation pharmaceutique effervescente de cefdinir ou d'un sel ou polymorphe pharmaceutiquement acceptable de celui-ci, comprenant au moins deux lubrifiants,
- l'un desdits lubrifiants étant le polyéthylène glycol et l'autre lubrifiant étant choisi parmi le fumarate de sodium et de stéaryle, la leucine, un poloxamère, le benzoate de sodium, le lauryl sulfate de sodium ou un mélange approprié de ceux-ci et le rapport en poids de cefdinir aux lubrifiants se situant dans la plage de 1:10 à 10:1, et
- ladite formulation ne comprenant pas de stéarate de magnésium.

2. - Formulation pharmaceutique selon la revendication 1, dans laquelle le rapport en poids de cefdinir aux lubrifiants se situe de préférence dans la plage de 1:10 à 8:1.

3. - Formulation pharmaceutique selon l'une quelconque des revendications précédentes, dans laquelle le rapport en poids de cefdinir aux lubrifiants se situe de façon davantage préférée dans la plage de 1:10 à 6:1.

4. - Formulation pharmaceutique selon l'une quelconque des revendications précédentes, dans laquelle la formulation est obtenue au moyen d'un procédé de compression de briquettes ne mettant pas en jeu de solvant liquide pendant l'étape de granulation.

5. - Formulation pharmaceutique selon l'une quelconque des revendications précédentes, dans laquelle la masse moléculaire moyenne de polyéthylène glycol se situe dans la plage de 7000 à 9000.

6. - Formulation pharmaceutique selon l'une quelconque des revendications précédentes, comprenant en outre un liant.

7. - Formulation pharmaceutique selon l'une quelconque des revendications précédentes, dans laquelle le liant est de préférence la polyvinylpyrrolidone.

8. - Formulation pharmaceutique selon l'une quelconque des revendications précédentes, dans laquelle la valeur K de la polyvinylpyrrolidone est de 30.

9. - Formulation pharmaceutique selon l'une quelconque des revendications précédentes, dans laquelle les lubrifiants sont de préférence respectivement le fumarate de sodium et de stéaryle et le polyéthylène glycol.

10. - Formulation pharmaceutique selon l'une quelconque des revendications précédentes, dans laquelle les lubrifiants sont respectivement un poloxamère et le polyéthylène glycol.

11. - Formulation pharmaceutique selon l'une quelconque des revendications précédentes, dans laquelle les lubrifiants sont respectivement le lauryl sulfate de sodium et le polyéthylène glycol.

12. - Formulation pharmaceutique selon l'une quelconque des revendications précédentes, comprenant en outre un désintégrant.

13. - Formulation pharmaceutique selon l'une quelconque des revendications précédentes, consistant en :
a. 50 à 90 % en poids de cefdinir ou d'un sel pharmaceutiquement acceptable de celui-ci ;
b. 5 à 30 % en poids d'acide citrique ;
c. 10 à 60 % en poids d'hydrogéno carbonate de sodium ;
d. 0,1 à 5 % en poids de polyvinylpyrrolidone ;
e. 0,1 à 5 % en poids de polyéthylène glycol ;
f. 0,1 à 3 % en poids de fumarate de sodium et de stéaryle ;
g. 1 à 5 % en poids d'arôme d'orange ;
h. 1 à 5 % en poids de sucralose.

14. - Formulation pharmaceutique selon l'une quelconque des revendications précédentes pour une utilisation dans la prévention ou le traitement de la bronchite, de la pharyngite, de l'otite moyenne, de la pneumonie, de la sinusite, de l'amygdalite, de la vaginose bactérienne et des maladies infectieuses des tissus chez les mammifères et en particulier chez les êtres humains.
